# EUROPEAN PATENT SPECIFICATION

(11) **EP 2 044 935 B1**
(45) Date of publication and mention of the grant of the patent: **05.01.2011**
(21) Application number: 07117778.6
(22) Date of filing: 02.10.2007
(51) Int. Cl.: A61K 31/05, A61K 31/352, A61K 36/185, A61P 29/00, A61P 35/00

(54) **A composition containing non-psychotropic cannabinoids for the treatment of inflammatory diseases**
Zusammensetzung mit nicht-psychotropen Cannabinoiden zur Behandlung von Entzündungserkrankungen
Composition contenant des cannabinoïdes non psychotropes pour le traitement de maladies inflammatoires

(43) Date of publication of application: 08.04.2009
(73) Proprietor: Vivacell Biotechnology Espana S.L., 14004 Cordoba (ES)
(72) Inventor: Muñoz Blanco, Eduardo, 14004, Córdoba (ES); Appendino, Giovanni, 14004, Córdoba (ES); Fiebich, Bernd L., 14004, Córdoba (ES); Grassi, Giampaolo, 14004, Córdoba (ES)
(74) Representative: Illescas, Manuel

(56) References cited:
- WO-A-99/52524
- US-B1- 6 403 126
- 7 REUNION ANUAL, SOCIEDAD ESPAÑOLA DE INVESTIGACION SOBRE CANNABINOIDES, TOLEDO, 23-25 NOVIEMBRE 2006, [Online] November 2006 (2006-11), pages 1-44, XP002469010 Retrieved from the Internet: URL:http://www.ucm.es/info/seic-web/libro2 006.pdf> [retrieved on 2008-01-30]
- EVANS A T ET AL: "CONSTITUENTS OF CANNABIS-SATIVA INHIBIT LIPOXYGENASE ACTIVITY" JOURNAL OF PHARMACY AND PHARMACOLOGY, LONDON, GB, vol. 37, 9 September 1985 (1985-09-09), page 43P, XP002114256 ISSN: 0022-3573
- BARRETT ML ET AL: "Cannflavin A and B, prenylated flavones from Cannabis sativa L" EXPERIENTIA, BIRKHAEUSER VERLAG. BASEL, CH, vol. 42, no. 4, 15 April 1986 (1986-04-15), pages 452-453, XP002114248 ISSN: 0014-4754
- ELSOHLY H ET AL: "CANNIPRENE A PROTOTYPE ANTI-INFLAMMATORY NATURAL PRODUCT" PLANTA MEDICA, THIEME, STUTTGART, DE, vol. 56, no. 6, 1990, pages 662-663, XP009095318 ISSN: 0032-0943
- BAEK S H ET AL: "BORON TRIFLUORIDE ETHERATE ON SILICA-A MODIFIED LEWIS ACID REAGENT (7). ANTITUMOR ACTIVITY OF CANNABIGEROL AGAINST HUMAN ORAL EPITHELOID CARCINOMA CELLS" ARCHIVES OF PHARMACAL RESEARCH, NATL. FISHERIES UNIVERSITY, PUSAN, KR, vol. 21, no. 3, June 1998 (1998-06), pages 353-356, XP000957635 ISSN: 0253-6269
- YANG KUO-CHING ET AL: "Molecular mechanisms of denbinobin-induced anti-tumorigenesis effect in colon cancer cells." WORLD JOURNAL OF GASTROENTEROLOGY : WJG 28 MAY 2005, vol. 11, no. 20, 28 May 2005 (2005-05-28), pages 3040-3045, XP002469127 ISSN: 1007-9327

## Description

The invention relates to a composition comprising at least one non-psycotropic cannabinoid and/or at least one phenolic or flavonoid compound and/or Denbinobin and their uses for the prevention and treatment of gastrointestinal inflammatory diseases and for the prevention and treatment of gastrointestinal cancers. It also relates to a phytoextract obtained from the plant *Cannabis sativa,* more particularly from the selected variety CARMA.

### BACKGROUND OF THE INVENTION

The therapeutic properties of the hemp plant, *Cannabis sativa,* have been known since antiquity, but the recreational use of its euphoric and other psychoactive effects due to the metabolite Δ-9-Tetrahydrocannabinoid (THC) has restricted for a long time its possible pharmaceutical application. THC is a potent agonist of the CB₁ receptors (Cannabinoid receptor type I) that is highly expressed in neuronal cells and is the responsible for mediating the psychotropic effects of the plant. THC has been also investigated against several types of cancer (Guzman M. Cannabinoids: potential anticancer agents. Nat Rev Cancer. 2003 Oct;3(10):745-55; Blazquez C, Casanova ML, Planas A, Del Pulgar TG, Villanueva C, Fernandez-Acenero MJ, Aragones J, Huffman JW, Jorcano JL, Guzman M. Inhibition of tumor angiogenesis by cannabinoids. FASEB J. 2003 Mar;17(3):529-31) and there is increasing evidence that THC may exert it anti-tumoral effects by both CB₁-dependent and -independent pathways (Kogan NM. Cannabinoids and cancer. Mini Rev Med Chem. 2005 Oct;5(10):941-52) indicating that other THC-analogues (synthetic or naturally occurring) lacking psychotropic activities may retain anti-tumoral activities (Ligresti A, Moriello AS, Starowicz K, Matias I, Pisanti S, De Petrocellis L, Laezza C, Portella G, Bifulco M, Di Marzo V. Antitumor activity of plant cannabinoids with emphasis on the effect of cannabidiol on human breast carcinoma. J Pharmacol Exp Ther. 2006 Sep;318(3):1375-87. *C. sativa* contains at least 400 chemical components, of which more than 60 have been identified to belong to the class of the cannabinoids (Stern E, Lambert DM. Medicinal chemistry endeavors around the phytocannabinoids. Chem Biodivers. 2007 Aug;4(8):1707-28).

On the other hand *Cannabis sativa* has been used historically for the treatment of diarrhoea and other gastrointestinal disorder. One explanation for such historical use is the fact that endocannabinoid system is widely distributed in the digestive system (Kulkarni-Narla A, Brown DR. Localization of CB1-cannabinoid receptor immunoreactivity in the porcine enteric nervous system. Cell Tissue Res. 2000 Oct;302(1):73-80). Besides, recent pharmacological studies have shown that THC inhibits gastrointestinal motility by activating CB₁ receptors in intestine submucose neurons (Mathison R, Ho W, Pittman QJ, Davison JS, Sharkey KA. Effects of cannabinoid receptor-2 activation on accelerated gastrointestinal transit in lipopolysaccharide-treated rats. Br J Pharmacol. 2004 Aug;142(8):1247-54). Some other experiments have shown that THC reduces intestinal inflammation in mice in which an intestinal inflammatory disease (IBD) has been induced (Massa F, Marsicano G, Hermann H, Cannich A, Monory K, Cravatt BF, Ferri GL, Sibaev A, Storr M, Lutz B. The endogenous cannabinoid system protects against colonic inflammation. J Clin Invest. 2004 Apr;113(8):1202-9). However, it is likely that many of the anti-inflammatory activities attributed to *Cannabis sativa* are mediated by compounds other than THC (Klein TW, Newton CA. Therapeutic potential of cannabinoid-based drugs. Adv Exp Med Biol. 2007; 601:395-413). Nevertheless the biological activities of a Cannabis-derived phytoextract containing non-psychotropic cannabinoids such as Cannabidiol (CBD) and Cannabigerol (CBG) and lacking THC have never been investigated.

In addition to cannabinoids other phenolic compounds such as Cannflavin A and B have been isolated from the plant Cannabis sativa (Barrett ML, Scutt AM, Evans FJ. Cannflavin A and B, prenylated flavones from Cannabis sativa L. Experientia. 1986 Apr 15;42(4):452-3). Cannflavin A hold potential anti-inflammatory activities since it can inhibit the release of prostaglandin E2 (Barrett ML, Gordon D, Evans FJ. Isolation from Cannabis sativa L. of cannflavin-a novel inhibitor of prostaglandin production. Biochem Pharmacol. 1985 Jun 1;34(11):2019-24). The pro-inflammatory prostaglandin E2 is produced by enzymatic activity of COX-2 that convert arachidonic acid into prostraglandins and other lipid mediators. The temporal association between loss of function of the tumor suppressor adenomatous polyposis coli (APC) and overexpression of cyclooxygenase 2 (COX-2) has been demonstrated in vivo and has led to the hypothesis that APC regulates COX-2 expression. Moreover there is evidence that Cox-2 inhibitors are chemopreventives agents that present colon cancer (Eisinger AL, Prescott SM, Jones DA, Stafforini DM. The role of cyclooxygenase-2 and prostaglandins in colon cancer. Prostaglandins Other Lipid Mediant 2007 Jan;82(1-4):147-54; Harris RE, Beebe-Donk J, Alshafie GA. Cancer chemoprevention by cyclooxygenase 2 (COX-2) blockade: results of case control studies. Subcell Biochem. 2007; 42:193-212).

Other documents disclose the pharmaceutical activity of different compounds isolates from *Canavis sativa,* like the anti-inflammatory effect of canniprene (EISohly H, Little TJr and ElSohly M. Canniprene: a prototype anti-inflammatory natural product. Planta Medica, Thieme, Stuttgart. DE 56(6):662-663), the anti-inflammatory effects of cannflavins A and B (Barrett ML, Scutt AM and Evans FJ. Cannflavin A and B, prenylated flavones from Cannabis sativa L. Experientia. 1986 Apr 15;42(4):452-3), the anti-inflammatory effects of cannflavin A, cannabigerol and cannabidiol (Evans A.T. et al., Constituents of Cannabis-sativa inhibit lipooxigense activity. Journal of Pharmacy and Pharmacology 37, page 43), or the anti-inflammatory effects of cannflavone-2 (cannflavin A) and of cannabinoids, including cannabigerol and cannabidiol, and also the use of said compounds for treating inflammatory diseases (WO 99/52524 A).

Phenanthrenequinones of non-terpenoide origin occur relatively rarely in the plant kingdom whereas oxygenated phenanthrenes are more widely distributed. Three major groups of 1,4-phenanthrenequinones may be distinguished according to their structure and biosynthesis. Representatives of the first group are hydroxy- and methoxysubstituted compounds such as annoquinone-A, cypripedine, denbinobine and combrestatin. (Krohn, K., Loocka, U., Paavilainena, K., Hausenb, B.M., Schmallec HW, and Kieseled H. Synthesis and electrochemistry of annoquinone-A, cypripedin methyl ether, denbinobin and related 1,4-phenanthrenequinones. ARKIVOC 2001 (i) 88-130). Interestingly, denbinobin have been shown to have anti-tumoral activities against leukaemia and colon cancer cell lines (Yang KC, Uen YH, Suk FM, Liang YC, Wang YJ, Ho YS, Li IH, Lin SY. Molecular mechanism of denbinobin-induced anti-tumarigenesis effect in colon cancer cells, World J Gastroenterol. 2005 May 28;11(20):3040-5, Huang YC, Guh JH, Teng CM. Denbinobin-mediated anticancer effect in human K562 leukemia cells: role in tubulin polymerization and Bcr-Abl activity. J Biomed Sci. 2005;12(1):113-21). Denbinobin was isolated for the first time from the orchid *Dendrobium nobile* (Lee YH, Park JD, Baek Nl, Kim Sl, Ahn BZ. In vitro and in vivo antitumoral phenanthrenes from the aerial parts of Dendrobium nobile. Planta Med 1995; 61: 178-180). However, 1,4-phenanthrenequinones could not be extracted from *Dendrobium nobile* cultivated at various places in Europe (Krohn, K., Loocka, U., Paavilainena, K., Hausenb, B.M., Schmallec HW, and Kieseled H. Synthesis and electrochemistry of annoquinone-A, cypripedin methyl ether, denbinobin and related 1,4-phenanthrenequinones. ARKIVOC 2001 (i) 88-130). More recently it has been shown that some Cannabis cultivars contain denbinobin, which can inhibit HIV-1 replication in T cells (Sánchez-Duffhues, G., Caballero, F.J., Calzado, M.A., Maxia, L., Appendino, G, Schmitz, L., and Muñoz E. El denbinobin aislado del Cannabis sativa es un potente inhibidor de la replicacion del VIH-1 por actuar sobre la ruta de NF-kB. 2006. 7a Reunion Anual. Sociedad Española de Investigación sobre Cannabinoides).

The relationship between inflammation and cancer was first suggested by Virchow in the 19^{th} century. A few years ago, however, real knowledge about this has been started to be known. Nowadays it is known that chronic infections and inflammations are a high risk factor for the development of tumours as it is the fact for 15 to 20% all cancers. One of the most important molecular mediators in inflammation is NF-kB (nuclear factor *kappa* B) transcription factor that is also involved in tumour promoting processes (Karin M, Greten FR. NF-kappaB: linking inflammation and immunity to cancer development and progression. Nat Rev Immunol. 2005 Oct;5(10):749-59). The activation of NF-kB in chronic inflammation is especially relevant as concern gastrointestinal carcinogenesis, and especially in colitis associated cancer (CAC) which represents up to 5% of all colonic cancer (Chung DC. 2000. The genetic basis of colorectal cancer: insights into critical pathways of tumorogenesis. Gastroenterology 119: 854-865). The cumulative incidence of CAC in patients with ulcerous colitis varies between 8 and 43% (Ekbom A. 1998. Risk of cancer in ulcerative colitis, J. Gastroenterol. Surg. 2: 312-313). NF-κB has been observed in the nucleus of macrophages in the lamina propia and in epithelial cells of biopsies of IBD and colorectal cancer patients (Boone DL, Lee EG, Libby S, Gibson PJ, Chien M, Chan F, Marlonia M, Burkett PR, Ma A. Recent advances in understanding NF-kappaB regulation. Inflamm Bowel Dis. 2002 May;8(3):201-12). It has also been shown an activation of NF-κB and an increase of Cox-2 expression in macrophages within polypomatous adenomes (Hardwick JC, van den Brink GR, Offerhaus GJ. van Deventer SJ, Peppelenbosch MP. NF-kappaB, p38 MAPK and JNK are highly expresses and active in the stroma of human colonic adenomatous polyps. Oncogene. 2001 Feb 15;20(7):819-27). COX-2 expression in infiltrated macrophages is considered as an initial profess in the evolution of colon carcinogenesis (Janne PA, Mayer RJ. Chemoprevention of colorectal cancer. N Engl J Med. 2000 Jun 29; 342(26):1960-8.).

Since NF-κB identification, it has been suggested that many of the proteins involved in its activation pathway, and hence responsible for inflammation and cancer, can be molecular targets for many drugs. Some of these drugs have been discovered so far, and some are being tested in clinical essays (Karin M, Yamamoto Y, Wang QM. The IKK NF-kappa B system a treasure trove for drug development. Nat Rev Drug Discov, 2004 Jan;3(1):17-26). Experimental models suggest that NF-κB inhibitors can be potentially active in acute intestinal inflammatory diseases (Taylor C, Jobin C.

Ubiquitin protein modification and signal transduction: implications for inflammatory bowel diseases. Inflamm Bowel Dis. 2005 Dec;11(12);1097-107). With the use of specific animal models it has recently been confirmed the role of NF-κB in the development of colorectal cancer of inflammatory origin (Greten FR, Eckmann L, Greten TF, Park JM, Li ZW, Egan LJ, Kagnoff MF, Karin M. IKKbeta links inflammation and tumorigenesis in a mouse model of colitis-associated cancer. Cell, 2004 Aug 6;118(3):285-96).

In this sense, Kuo-Ching Yang *et al*., 2005 (Kuo-Ching Yang, Yih-Huei Uen, Fat-Moon Suk, Yu-Chih Liang, Ying-Jan Wang, Yuan-Soon Ho, I-Hsuan Li, Shyr-Yi Lin. Molecular mechanisms of denbinobin-induced antitumorigenesis effect in colon cancer cells. World J Gastroenterol 2005;11(20):3040-3045) teaches the ability of denbinobin to inhibit colon cancer growth, further reporting the know anti-inflammatory effects thereof. Seung-Hwa Baek and Chang-Joon Lee (Boron trifluoride etherate on alumina-modified lewis acid reagent(III): Synthesis of 5-alkyl-3-(1-thioxolanyl-cyclohexenyl)-resorcinol derivatives. *Archives of Pharmacal Research* 15(1)) discloses the ability of cannabigerol and cannabidiol to inhibit the growth of epitheloid carcinoma cells. US 6403126 B1 describes a method of extracting cannabinoids, cannflavins, and/or essential oils from hemp and/or of producing a whole hemp extract lacking Δ⁹-THC (Δ⁹-tetrahydrocannabinol), a pharmaceutical composition comprising the extract prepared by said method, and also indicates the beneficial use of cannabinoids and cannflavins as anti-inflammatory and antineoplastic agents. Sánchez-Duffhues *et al*., 2006 (7^{a} REUNIÓN ANUAL. SOCIEDAD ESPANOLA DE INVESTIGACIÓN SOBRE CANNABINOIDES, TOLEDO, page 34, http://www.ucm.es/info/seic-web/documentos/libro2006.pdf) describes the antiinflamatory activity of the extract LMX-252 from *Cannabis sativa,* indicating that said extract contains cannabigerol, cannabidiol, cannabinochromene, canniprene, cannabispiranol, cannflavin-A, denbinobin and some THC. It also teaches the ability of said extract to inhibit the NF-kB pathway, indicating that denbinobin may be the agent responsible for this effect.

### SUMMARY OF THE INVENTION

The present invention relates to a composition comprising cannabidiol and denbinobin, and having a content of delta-9-tetrahydrocannabidiol less than 0.7% of the total weight of the composition, wherein
a. denbinobin is present at concentrations between 0.25 µM and 0.5 µM when cannabidiol is present at concentrations between 10 µM and 20 µM and
b. denbinobin is present at concentrations between 0.25 µM and 1 µM when cannabidiol is present at concentrations of 10 µM.

The present invention also relates to the use of said composition for the manufacture of a medicament for the prevention and/or treatment of inflammatory diseases.

Described herein is a composition of at least one non-psycotropic cannabinoid compound and/or at least one phenolic or flavonoid compound and/or the 1,4- phenanthrenequinone Denbinobin and its use for the prevention and treatment of gastrointestinal inflammatory and/or cancer diseases.

Thus, this composition comprises specific combinations of at least one non-psycotropic cannabinoid compound selected, but not limited to, from the list comprising cannabigerol or cannabidiol and/or at least one phenolic or flavonoid compound selected from the list comprising canniprene, cannabispiranol, canflavin-A or canflavin-B; and Denbinobin. This composition may also contain other compounds; however, if tetrahydrocannabinoid (delta-9-tetrahydrocannabidiol or THC) is present in the composition, its content is less than 0,7% of the total weight of the composition.

Composition comprising cannabigerol and cannabidiol may be in a ratio between 5:1 and 1:1 respectively, or in a ratio 4:1 or 3:1. The composition further comprises canflavin-A and denbinobin, which are within the range, of cannabigerol 20-45%, cannabidiol 2-15%, canflavin-A 1-5% and denbinobin 0,1-1% in respect of the total weight of the composition. Compositions may be also within the range, of cannabigerol 30-35%, cannabidiol 6-10%, canflavin-A 2-4% and denbinobin 0,4-0,7% respectively.

Other cannabinoids such as Cananabichromene (CBC) and Carmagerol (dyhydroxy-CBG) and other stilbenoids such as Canniprene, Cannabispiranol, Cannabispirane may also be contained in the composition.

Some of the compounds cited above, in particular cannabigerol, cannabidiol, canniprene, cannabispiranol, canflavin-A, denbinobin and cannabispirane present the following structures:

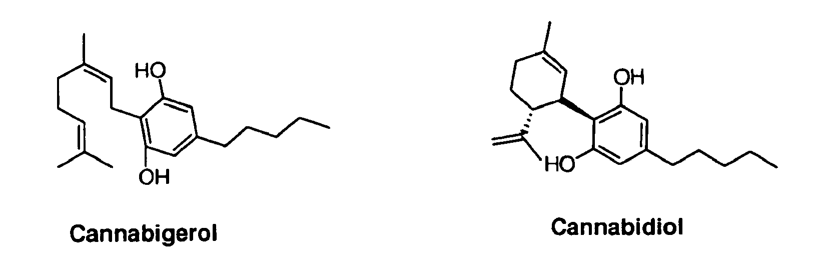

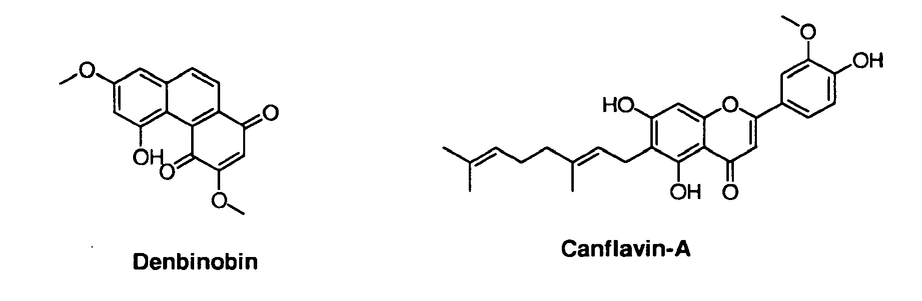

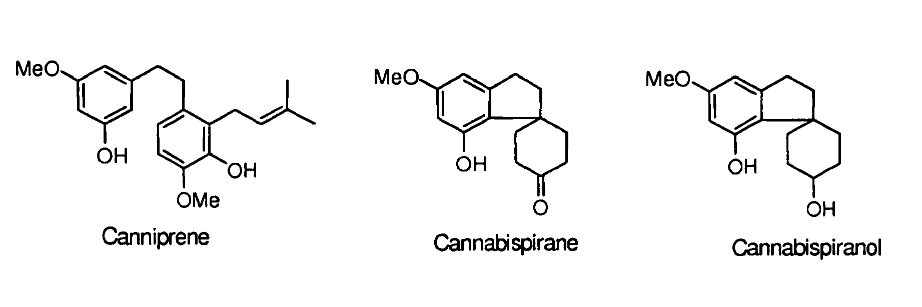

The cannabigerol, cannabidiol, canflavin-A and denbinobin of these compositions could be extracted from at least one extract from at least one cannabis plant. Thus, the present invention also relates to a cannabis-based composition that included specific combinations of at least one non-psycotropic cannabinoid compound and/or at least one phenolic or flavonoid compound and/or the 1,4- phenanthrenequinone Denbinobin.

The term "cannabis-based composition" as used herein refers to an acetonic or partitioned plant extract containing the bioactive compounds referred in the present invention.

The cannabis plant is selected from *Cannabis sativa* and more preferably from the variety CARMA of *Cannabis sativa.*

Thus, the present invention provide an herbal therapy for the prevention and treatment of gastrointestinal inflammatory and cancer diseases which employs specific combinations of the non-psychotropic cannabinoids Cannabigerol and Cannabidiol, the flavonoid Canflavin A and the 1,4-phenanthrenequinone Denbinobin that are provided by either an acetone extract or by a partitioned extract isolated from the selected *Cannabis Sativa* variety denominated CARMA.

Using specific and suitable models for inflammation the present invention provide evidence showing that these compositions have potent anti-inflammatory activity "in vivo" and "in vitro" due to the synergistic or additive effects of the compounds contained in the standardized extract.

The present invention further provides that the CARMA-derived extract is cytotoxic for gastrointestinal cell lines and shows protective effect against Azoxymethane-induced colon cancer and angiogenesis in mammalians. Typical dosing protocols for the combination therapy are provided but not restricted. Various other objects and advantages of the present invention will become apparent to one skilled in the art from the drawings and the following description of the invention.

The CARMA-derived extract and the compounds Cannabigerol, Cannabidiol, Canflavin-A and Denbinobin (alone or in combination) inhibit pro-inflammatory events that are involved in the physiopathology of intestinal inflammation. This finding is essential for the formulation of the pharmaceutical preparation of a non-psychotropic herbal therapy based on *Cannabis Sativa* with minimal adverse toxicological properties.

Therefore, the present invention also refers to the use as a medicament of a composition comprising specific combinations of at least one non-psycotropic cannabinoid compound and/or at least one phenolic or flavonoid compound and/or the 1,4-phenanthrenquinone denbinobin,as described above. Preferably, the uses of these compositions are for the manufacture of a medicament for the prevention and/or treatment of inflammatory diseases or for the prevention and/or treatment of cancer.

Throughout the description and claims the word "comprise" and its variations are not intended to exclude other technical features, components, or steps. Additional objects, advantages and features of the invention will become apparent to those skilled in the art upon examination of the description or may be learnt by practice of the invention. The following examples and drawings are provided by way of illustration, and are not intended to be limiting of the present invention.

### BRIEF DESCRIPTION OF THE DRAWINGS

FIG. 1 shows the HPLC profile content of cannabinoids (CBG+CBD), Canflavin A, Canniprene, Cannabispiranol and denbinobin in an acetone extract from the *Cannabis sativa* (variety CARMA-CBG).
FIG. 2 shows the inhibitory effect of the *Cannabis sativa* (variety CARMA-CBG) extract on TNFa-induced NF-κB transcriptional activity.
FIG. 3 shows the inhibitory effect of the *Cannabis sativa* (variety CARMA-CBG) extract on the NF-kB binding activity to DNA.
FIG. 4 shows the effect of the *Cannabis sativa* (variety CARMA-CBG) extract on the biochemical NF-kB signalling pathways.
FIG. 5 shows the effect of the *Cannabis sativa* (variety CARMA-CBG) extract on the release of IL-1β in LPS-activated human monocytes.
FIG. 6 shows the effect of the *Cannabis sativa* (variety CARMA-CBG) extract on the release of TNFα in LPS-activated human monocytes.
FIG. 7 shows the cytotoxic effects of the *Cannabis sativa* (variety CARMA-CBG) extract on gastrointestinal cancer cell lines.
FIG. 8 shows the protective effects of the *Cannabis sativa* (variety CARMA-CBG) extract given orally on dextran sulphate-induced colon inflammation in mice (macroscopic score).
FIG. 9 shows the protective effects of the *Cannabis sativa* (variety CARMA-CBG) extract given orally on dextran sulphate-induced colon inflammation in mice (histological evaluation).
FIG. 10 shows the effects *Cannabis sativa* (variety CARMA-CBG) extract given orally on weight loss in azoxymethane/DSS-induced colon cancer disease in mice.
FIG. 11 shows the protective effects of the *Cannabis sativa* (variety CARMA-CBG) extract given orally on azoxymethane/DSS-induced colon cancer disease in mice (A; Mortality rate; B; Number of colonic tumours in individual animals).
FIG. 12 shows the anti-angiogenic effect of the *Cannabis sativa* (variety CARMA-CBG) extract given orally in matrigel injected mice.
FIG. 13 shows the effects of Cannabigerol, Cannabidiol, Denbinobin or Canflavin-A on TNFα-induced NF-kB activation.
FIG. 14 shows the effects of Cannabigerol, Cannabidiol, Denbinobin or Canflavin-A on LPS-induced IL-1β release.
FIG. 15 shows the effects of Cannabigerol, Cannabidiol, Denbinobin or Canflavin-A on LPS-induced TNFα release.
FIG. 16 shows the cytotoxic effects of Cannabigerol and Cannabidiol on gastrointestinal cancer cell lines

### DETAILED DESCRIPTION OF THE INVENTION

The *Cannabis sativa* variety CARMA has been obtained using Italian monoecious material (South Italy) combined with dioecious variety (Carmagnola). The principal distinctive characters of this new variety are the chemotype stable and unique and the monoecious character combined with fibre quality and shape of traditional Italian dioecious variety. The cannabinoid composition of this variety is the same for all the plants. They produce Cannabigerol (CBG) and Cannabidiol (CBD). In the average, the 95% of total cannabinoids content are CBG and CBD and very limited concentration of delta-9-tetrahydrocannabinol (THC) is present. Optimal sowing date: 10 of April, in Italy. Harvest time for plant production is the beginning of August and seed harvest could be done at the middle of September.

Breeding scheme (indicate female component at the first place): 1998-Carmagnola (single plant CAR-Y) crossed with monoecious plant of South Italy genotype (F1). All plants with Cannabigerol plus Cannabidiol chemotype.1998- Carmagnola (CAR-Y) pollinated with monoecious F1 plants (first back cross, BC1). 1999- Carmagnola (CAR-Y) pollinated with monoecious BC1 (second back cross, BC2). 1999- Carmagnola (CAR-Y) pollinated with monoecious BC2 (third back cross, BC3). 2000-Carmagnola (single super plant) pollinated with 20 BC3 monoecious plants. 2001- Mass production of R1 of the variety in isolated field condition with morphological and chemical selection. 2002- Mass production of R2 of the variety in isolated field condition with morphological and chemical selection (CARMA-CBG variety).

### CARMA-CBG extracts production.

A dried, powdered plant material (flowerheads, 850 g) is heated in an oven at 120°C for 2 h. After cooling, the decarboxylated plant material is extracted with acetone (3 x 5 L, 1 h each). The pooled acetone extracts are evaporated, to afford a dark brown gum (82 g). The crude extract has a ratio of approximately 8:1 between cannabinoids and the phenolics. For the partitioned extract primary acetone extract has been partitioned between hexane and aqueous methanol and contains a ratio of approximately 1:1 between cannabinoids and the phenolics. The yield production of extracts was 4 to 8% of the dried plant material.

The content of cannabinoids in the CARMA-CBG acetone extract was: 1) Cannabigerol (CBG): 35-45%; 2) Cannabidiol (CBG): 3-10%; 3) Δ-9-Tetrahydrocannabinoid (THC): 0.0-0.7%; and 4) Carmagerol (dihydroxy-CBG): 0.2- 2%.

The content of phenolic compounds in the CARMA-CBG acetone extract was: 1) Canflavin A: 2-4%; 2) Canflavin B: 1-2%; 3) Canniprene: 4-5%; 4) Cannabispiranol: 0.5- 2%; 5) Cannabispiranone: 1- 4%.

The content of 1,4-phenanthrenquinones in the CARMA-CBG acetone extract was: Denbinobin 0.2-1 %

### EXAMPLES

The following examples are provided by way of illustration only and not by way of limitation. Those of skill in the art will readily recognize a variety of noncritical parameters that could be changed or modified to yield essentially similar results.

### EXAMPLE 1: HPLC characterization of the profile content of cannabinoids (CBG+CBD), Canflavin A, Canniprene, Cannabispiranol and denbinobin in an acetonic extract from the Cannabis sativa (variety CARMA-CBG).

1 g of powdered plant material was exhaustively extracted with acetone. The extract was partitioned between water-methanol (9:1, 1 mL) and hexane (4 mL). The lower methanolic phase was evaporated and dissolved in methanol (0.2 mL) and analyzed by RP-HPLC on a Symmetry C-18 column (5 micron, 4.6 x 150 mm, Waters), using the following conditions:
Detection: UV (210 and 272nm)
Flow: 1 ml/min
Solvent A: 0.5%v/v Orthophosphoric acid in Water
Solvent B: Acetonitrile

| | | | | | | |
|---|---|---|---|---|---|---|
| Gradient: | Time | 0 | 8 | 14 | 24 | 30 |
| | %B | 40 | 40 | 50 | 90 | 99 |

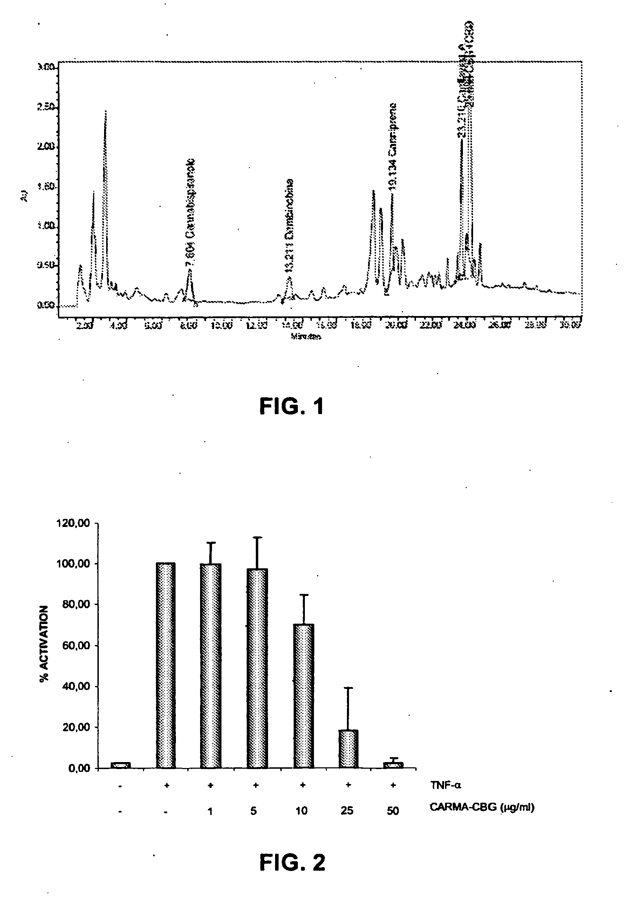

See Figure 1 (FIG.1) and Table 1

**Table 1**

| | RT (min) | Area (V*sec ) | % Area | Height (V) | % Height |
|---|---|---|---|---|---|
| 1 | 7.604 | 6103673 | 11.27 | 395711 | 6.88 |
| 2 | 13.211 | 4581869 | 8.46 | 279994 | 4.87 |
| 3 | 19.134 | 7916718 | 14.61 | 1011006 | 17.58 |
| 4 | 23.210 | 10823021 | 19.98 | 1738142 | 30.23 |
| 5 | 23.630 | 24746110 | 45.68 | 2324432 | 40.43 |

### EXAMPLE 2. Isolation and structures determination of the biologically active compounds isolated from Cannabis sativa (variety CARMA-CBG).

The plant material (200g) was heated in an oven at 120°C for two hours. After cooling, it was exhaustively extracted with acetone to afford a dark-black residue (16.4 g) that was dissolved in methanol (70 mL) and filtered over 40 g of RP18 silica gel. The filtration bed was washed with further 50 mL of methanol, and the pooled filtrated were evaporated, to afford 11.8 g of residue. This was fractionated by gravity column chromatography on silica gel to afford four subfractions (A-D). Subfraction A was crystallized by hexane to afford 4.70 g CBG as a white powder. The mother liquors were crystallized twice from hexane-methanol to afford 230 mg CBD. Subfraction B was crystallized from ether to afford 10 mg denbinobine.The mother liquors were purified by prep. HPLC (hexane-Ethyl acetate 7:3) to afford 85 mg canniprene, further 12 mg denbinobin and 21 mg cannflavin A. Subfraction C was crystallized from ether to afford 18 mg cannbispiranol and a mixture of cannflavin A and B, further separated by HPLC to afford 12 mg cannflavin A and 8 mg cannflavin B. Subfraction D was purified by prep. HPLC (hexane-Ethyl acegtate 5:5) to afford 12 mg dihydroxycannabigerol (= carmagerol), and 16 mg cannabispiranone.

### EXAMPLE 3. The CARMA-CBG extract inhibits TNFα-induced NF-kB transcriptional activity.

This example demonstrates the in vitro effect of the present inventive method by illustrating the inhibition by CARMA-CBG extract on the NF-kB-dependent gene transcriptional activity.

The potency of CARMA-CBG extract in inhibiting NF-kB-dependent transcriptional activity was assayed in a Jurkat-LTR-Luc cell line. The Jurkat-5.1 cell line is a T cell line stably transfected with a plasmid containing the luciferase gene driven by the HIV-1-LTR promoter. This cell line is highly responsive to TNF-α, which activated the classical NF-kB pathway. Therefore the pro-inflammatory cytokine TNFα induces the NF-kB-dependent transcriptional activity of the HIV-LTR promoter (Sancho R, Calzado MA, Di Marzo V, Appendino G, Munoz E. Anandamide inhibits nuclear factor-kappaB activation through a cannabinoid receptor-independent pathway. Mol Pharmacol. 2003 Feb;63(2):429-38). This cellular model have been widely used for the screening of natural and synthetic NF-kB inhibitors ( Appendino G, Ottino M, Marquez N, Bianchi F, Giana A, Ballero M, Sterner O, Fiebich BL, Munoz E. Arzanol, an anti-inflammatory and anti-HIV-1 phloroglucinol alpha-Pyrone from Helichrysum italicum ssp. microphyllum. J Nat Prod. 2007 Apr;70(4):608-12; Appendino G, Maxia L, Bascope M, Houghton PJ, Sanchez-Duffhues G, Munoz E, Sterner O. A meroterpenoid NF-kappaB inhibitor and drimane sesquiterpenoids from Asafetida. J Nat Prod. 2006 Jul;69(7):1101-4; Marquez N, Sancho R, Bedoya LM, Alcami J, Lopez-Perez JL, Feliciano AS, Fiebich BL, Munoz E. Mesuol, a natural occurring 4-phenylcoumarin, inhibits HIV-1 replication by targeting the NF-kappaB pathway. Antiviral Res. 2005 Jun;66(2-3):137-45) Jurkat 5.1 cells were preincubated for 30 min with increasing concentrations of CARMA-CBG extract dissolved in DMSO followed by stimulation with TNF-α (2 ng/ml) for 6 h. Cells were washed twice in PBS and lysed in 25 mM Tris-phosphate, pH 7.8, 8 mM MgCl₂, 1 mM DTT, 1% Triton X-100, and 7% glycerol for 15 min at room temperature. Then the lysates were spun down and the supernatant was used to measure luciferase activity using an Autolumat LB 9510 (Berthold Technologies). Protein concentration was determined by the Bradford method (Bio-Rad, Richmond, CA). The results are represented as the percentage of activation (considering the 100% of activation the values pf R.L.U. obtained with TNF-α treated cells in the absence of CARMA-CBG extract). Results represent mean ± SD of three different experiments and shows that the CARMA-CBG extract, in a concentration-dependent manner, inhibited TNFα-induced NF-kB transactivation (Figure 2).

### EXAMPLE 4. The CARMA-CBG extract inhibits TNFα-induced NF-kB binding to DNA activity.

This example demonstrates the in vitro effect of the present inventive method by illustrating the inhibition by CARMA-CBG extract on the NF-kB-DNA binding activity.

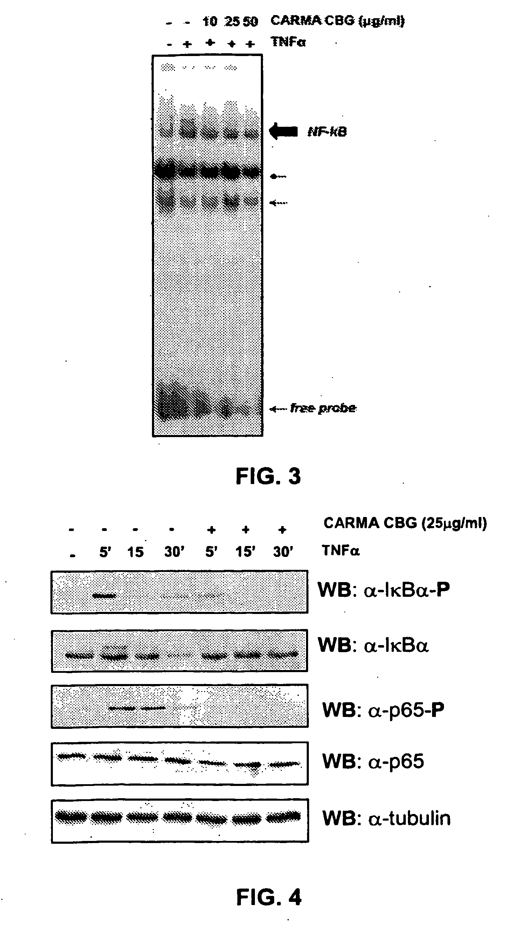

Inhibition of the NF-kB transcription factor binding to DNA was assayed by electrophoretic mobility shift assays (Sancho R, Calzado MA, Di Marzo V, Appendino G, Munoz E. Anandamide inhibits nuclear factor-kappaB activation through a cannabinoid receptor-independent pathway. Mol Pharmacol. 2003 Feb;63(2):429-38). Jurkat-5.1 cells were stimulated with the TNFα in the absence or the presence of increasing concentrations of the CARMA-CBG extract dissolved in DMSO. Cells were then washed twice with cold PBS and proteins from nuclear extracts isolated. Protein concentration was determined by the Bradford method. For the electroforetic mobility shift assay, a consensus oligonucleotide probes NF-kB, was end-labelled with [γ-³²P]ATP. The binding reaction mixture contained 3 µg of nuclear extract, 0.5 µg poly(dl-dC), 20 mM Hepes pH 7, 70 mM NaCl, 2 mM DTT, 0.01 % NP-40, 100 µg/ml BSA, 4% Ficoll, and 100,000 cpm of end-labelled DNA fragments in a total volume of 20 µl. After 30 min incubation at 4 °C, the mixture was electrophoresed through a native 6% polyacrylamide gel containing 89 mM Tris-borate, 89 mM boric acid and 1 mM EDTA. Gels were pre-electrophoresed for 30 min at 225 V and then for 2 h after loading the samples. These gels were dried and exposed to X-Ray film at -80 °C. It is shown that in Jurkat T cells the extract CARMA-CBG induce a dose-related decrease on TNFα-induced NF-κB binding activity (Figure 3).

### EXAMPLE 5. The CARMA-CBG extract inhibits TNFα-induced IkBα degradation and p65 phosphorylation.

This example demonstrates the in vitro effect of the present inventive method by illustrating the inhibition by CARMA-CBG extract on the biochemical signalling pathways that activates the classical NF-kB pathway.

The inhibitory effects of CARMA-CBG on TNFα-induced IkBα phosphorylation and degradation and on p65 (serine 536) phosphorylation was studied by inmunoblots. Jurkat-5.1. cells were stimulated with TNFα (2 ng/ml) during 5, 15 or 30 min in the absence of the presence of CARMA-CBG extract (25 µg/ml). Cells were then washed with PBS and proteins extracted from cells in 50 µl of lysis buffer (20 mM Hepes pH 8.0, 10 mM KCI, 0.15 mM EGTA, 0.15 mM EDTA, 0.5 mM Na₃VO₄, 5 mM NaFl, 1 mM DTT, leupeptin 1 µg/ml, pepstatin 0.5 µg/ml, aprotinin 0.5 µg/ml, and 1 mM PMSF) containing 0.5% NP-40. Protein concentration was determined by the Bradford method and 30 µg of proteins were boiled in Laemmli buffer and electrophoresed in 10% SDS/polyacrylamide gels. Separated proteins were transferred to nitrocellulose membranes (0.5 A at 100 V; 4 °C) for 1 h. The blots were blocked in TBS solution containing 0.1 % Tween 20 and 5% nonfat dry milk overnight at 4 °C, and immunodetection of IκBα, phosphor-IκBα, total p65, phospho-p65 and tubulin was carried out with specific mAbs and HRP-labelled secondary antibody using an ECL system (Figure 4).

### EXAMPLE 6. The CARMA-CBG extract inhibits the release of IL-1β in LPS-stimulated human monocytes.

This example demonstrates the in vitro effect of the present inventive method by illustrating the inhibitory effects of the CARMA-CBG extract on the release of the pro-inflammatory cytokine IL-1β by LPS-stimulated monocytes.

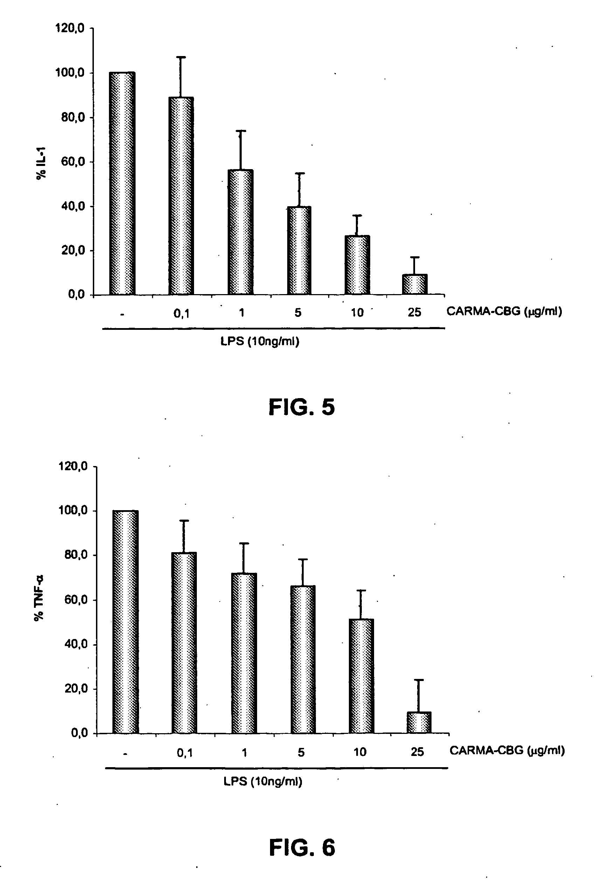

The inhibitory effect of CARMA-CBG extract on IL-1β release was studied in human peripheral mononuclear phagocytic cells. Monocytes from healthy human donors were prepared following a standardised protocol (Ficoll gradient preparation) using a completely endotoxin-free cultivation. Using 50 ml tubes, 25 ml Ficoll were loaded with 25 ml blood of buffy coats from healthy blood donors. The gradient was established by centrifugation at 1,800 r.p.m., 20° C for 40 min by using slow acceleration and brakes. Peripheral blood mononuclear cells in the interphase were carefully removed and re-suspended in 50 ml pre-warmed phosphate buffered saline followed by centrifugation for 10 min at 1,600 r.p.m. and 20° C. The supernatant was discarded and the pellet washed in 50 ml PBS and centrifuged as described above. The pellet was then re-suspended in 50 ml RPMI-1640 low endotoxin-medium supplemented with 10% human serum (PAA). After counting the amount of cells in a particle counter, cells were seeded in 24-well plates for ELISA and incubated at 37°C/5% CO₂. The medium and the non-adherent cells (lymphocytes) were removed and fresh RPMI-1640 medium containing 1% human serum was added. The monocytes were treated with LPS (10 ng/ml), in the absence or presence of increasing concentrations of CARMA-CBG extract dissolved in DMSO for 24 h. The production of the IL-1β was determined by ELISA. The extract significantly inhibited in a concentration-dependent manner the release of IL-1β in LPS-stimulated cells. LPS-mediated activation in the absence of CARMA-CBG was arbitrarily set as 100% of IL-1β release (Figure 5).

### EXAMPLE 7. The CARMA-CBG extract inhibits the release of TNFα in LPS-stimulated human monocytes.

This example demonstrates the in vitro effect of the present inventive method by illustrating the inhibitory effects of the CARMA-CBG extract on the release of the pro-inflammatory cytokine TNF-α by LPS-stimulated monocytes.

The inhibitory effect of CARMA-CBG extract on TNF-α release was studied in human peripheral mononuclear phagocytic cells. Monocytes were isolated as in Example 6 and were treated with LPS (10 ng/ml), in the absence or presence of increasing concentrations of CARMA-CBG extract dissolved in DMSO for 24 h. The production of the TNFα was determined by ELISA. The extract significantly inhibited in a concentration-dependent manner the release of TNF-α in LPS-stimulated cells. LPS-mediated activation in the absence of CARMA-CBG was arbitrarily set as 100% of TNF-α release (Figure 6).

### EXAMPLE 8. The CARMA-CBG extract induce cytotoxicity in the gastrointestinal cancer cell lines AGS and HCT-116

This example demonstrates the in vitro anti-tumoral effects of the present inventive method by illustrating the cytotoxic effects of the CARMA-CBG extract on the AGS (gastric cancer) and HCT-116 (Colon cancer) tumoral cell lines.

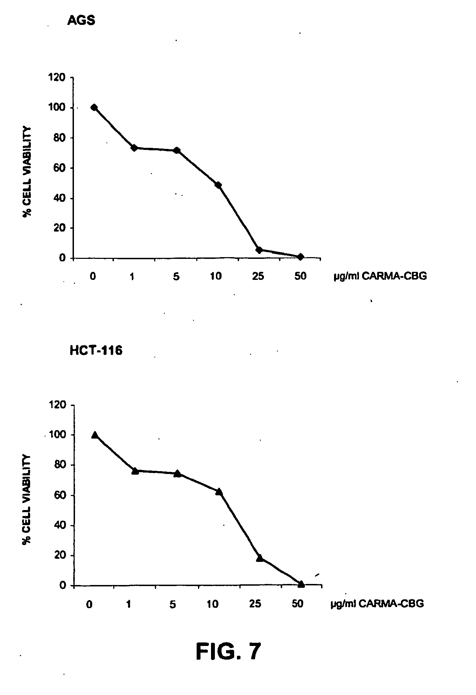

The cytotoxic effect of the CARMA-CBG extract was investigated by the MTT assay. Briefly, AGS and HTC116 cells were cultivated at a density of 1.0 × 10⁴ cells/ml in 96-well plates, 100 µl cell suspension per well and cultured in DMEM containing 10% fetal calf serum for 12 h. Cells were treated with or without increasing concentrations of the CARMA-CBG extract for 24 h. After that 50 µl of MTT (5 mg/ml) from a mixture solution of MTT:DMEM (1:2) was added to each well, and cells were incubated for 4 h at 37° C in darkness, and then reaction stopped, supernatant removed and 100 µl DMSO added to each well for 10 min, in gentle shaking. Finally the absorbance was measured at 570 nm using a multifunctional microplate reader (TECAN GENios Pro). The extract significantly decreased the percentage of viability that was assigned 100% to untreated cells (Figure 7).

### EXAMPLE 9. Protective effects of the CARMA-CBG extract on DSS-induced inflammatory bowel disease (macroscopic evaluation).

This example demonstrates "in vivo" the anti-inflammatory effects of the present inventive method by illustrating that oral treatment with CARMA-CBG extract prevents the onset of the chemically induced colon inflammatory disease in Swiss mice. The dextran sodium sulfate (DSS)-induced inflammatory bowel disease (IBD) is a murine model that has been shown to mimic some of the pathologies seen in ulcerative diseases (Neurath MF, Fuss I, Schurmann G, Pettersson S, Arnold K, Muller-Lobeck H, Strober W, Herfarth C, Buschenfelde KH. Cytokine gene transcription by NF-kappa B family members in patients with inflammatory bowel disease. Ann N Y Acad Sci. 1998 Nov 17;859:149-59).

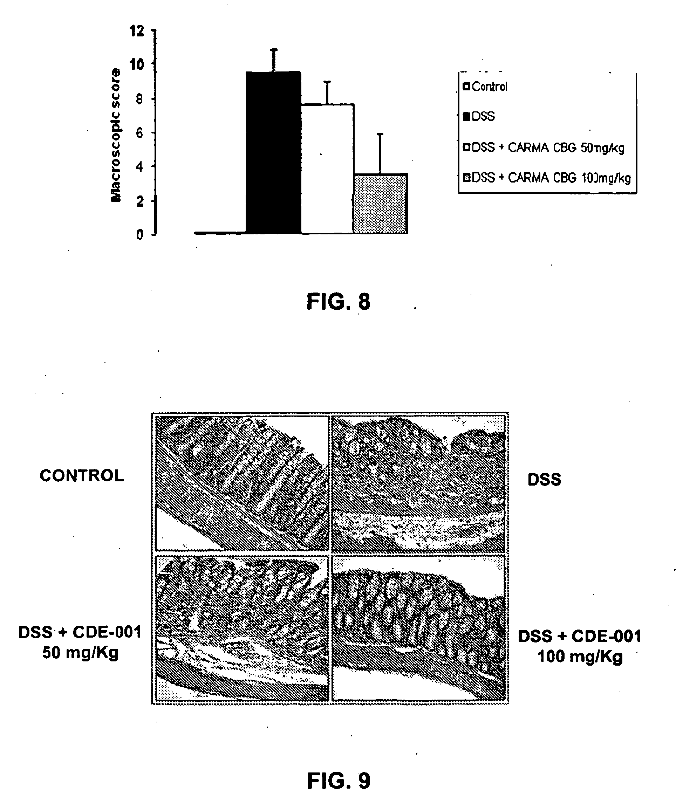

Swiss mice (Control Group, n=7) and CARMA-CBG mice (Two CBG Groups, n=6) were given 3% (w/v) DSS in their drinking water (MW: 36000-50000, ICN Pharmaceuticals Inc., Calif., USA) for 7 days. From day to 1 to 7 the CARMA-CBG groups were given orally either 50 mg/Kg or 100 mg/Kg once a day. The CARMA-CBG extract was dissolved in a saline solution containing 10% Chremophor. After finishing DSS administration, the mice were sacrificed by ether anesthesia, and the colons were removed to examine colitis. The final disease score were a combination of three different scores: 1) Stool score; 0 = normal; 1= loosey shape pellets; 2= amorphous, moist pellets; 3= diarrhoea (Presence of blood add 1 point): 2) Colon length score; 0 = <5% shortening; 1= 5-14% shortening; 2= 15-24% shortening; 3= 25-35% shortening; 4= >35% shortening: 3) Colon damage score: 0 = normal; 1= mild inflammation; 2= more widely distributed inflammation ; 3= extensively widely distributed inflammation (Figure 8).

### EXAMPLE 10. Protective effects of the CARMA-CBG extract on DSS-induced inflammatory bowel disease (histological evaluation).

This example demonstrates "in vivo" the anti-inflammatory effects of the present inventive method by illustrating that oral treatment with CARMA-CBG extract prevents the onset of the chemically induced colon inflammatory disease in Swiss mice. The colonic tissue from controls, DSS-elicited animals untreated or treated orally with the CARMA-CBG extract were fixed with paraformaldehyde (4%). Sagital sections of 6 µM were performed with a microtome and the preparations stained with Haematoxylin-eosin. Colons from DDS-treated mice showed an extensive epithelial damage accompanied by transmural infiltration of inflammatory cells. In contrast, in the mice treated orally with CARMA-CBG during the time of DSS induction of IDB the glandular epithelium was highly conserved and few inflammatory cells were identified in the lamina propia (Figure 9).

### EXAMPLE 11. Antitumoral effect of the CARMA-CBG extract on given orally on azoxymethane-induced colon cancer disease in mice.

This example demonstrates "in vivo" the anti-tumoral effects of the present inventive method by illustrating that oral treatment with CARMA-CBG extract greatly prevents Colitis Associated Cancer (CAC) in mice. The animal treatment with a single injection of chemical mutagen azoxymethane followed by oral treatment with dextran sodium sulphate is a murine model that has been shown to mimic colon carcinogenesis in humans (Suzuki R, Kohno H, Sugie S, Nakagama H, Tanaka T. Strain differences in the susceptibility to azoxymethane and dextran sodium sulfate-induced colon carcinogenesis in mice. Carcinogenesis. 2006 Jan;27(1):162-9).

6-8 week-old mice C57BL/6J were used in this study. All animals were housed in plastic cages (5 or 6 mice/cage), with free access to drinking water and a pelleted basal diet, under controlled conditions of humidity, light (12/12 hr light/dark cycle) and temperature (23-26° C). They were randomized by body weight into experimental and control groups. A colonic carcinogen Azoxymethane (AOM) was purchased from Sigma Chemical Co. Mice were injected intraperitoneally (i.p.) with 12.5 mg/kg AOM. After five days of the injection, 2.5% DSS (MW 36 - 50 kDa) was given in the drinking water over five days, followed by 16 days of regular water. This cycle was repeated twice (five days of 2.5% DSS and four days of 2% DSS) and mice were sacrificed ten days after the last cycle.

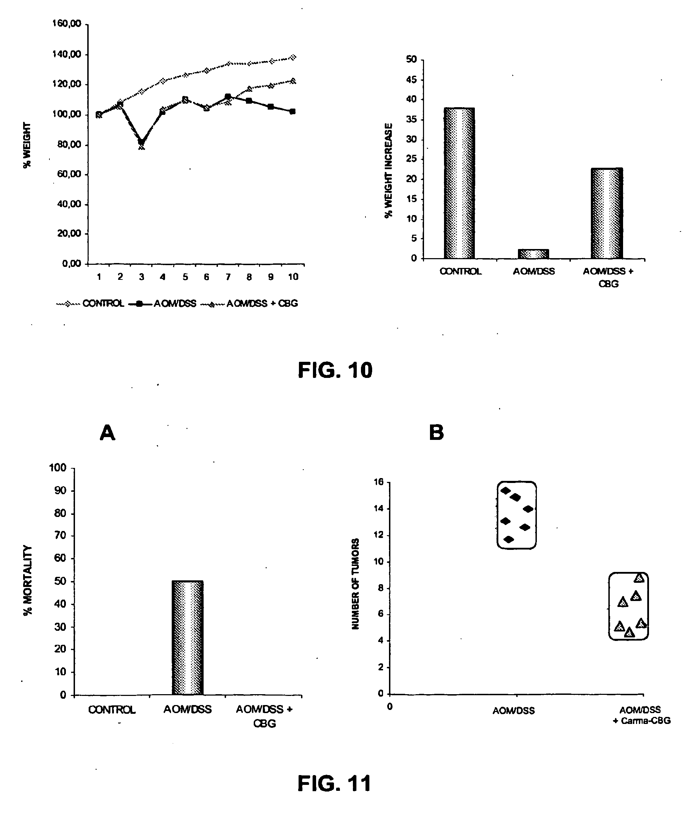

The CARMA-CBG extract dissolved in saline solution containing 10% Cremophor was given orally to the indicated group during the DSS administration periods, the treatment was given daily, and in the resting periods, the extract was administrated three times per week. Mice were sacrificed ten days after the last cycle. The whole intestinal tract of each mouse was removed, rinsed gently in PBS using a syringe and opened lengthwise. Tumours counts were performed in a blinded fashion and our data show that animals treated with the CARMA-CBG extract developed and average of 6 tumours per animal compared with there control group where the average was 16 tumours per animal (Figure 11 B)

Weight controls were performed also weekly for every group. Increase of body weight was observed in control group during the whole process. Loss of weight was observed during the first DSS period in both groups that received it. After the sixth week of treatment a significant recovering in the CARMA-CBG-treated group was observed, comparing to the non-treated group receiving also AOM/DSS (Figure 10).

Finally the mortality rate was reduced from 50% in control group (AOM+DSS in the absence of CARMA-CBG) to 0% in CARMA-CBG treated group (Figure 11A)

### EXAMPLE 12. In vivo anti-angiogenic effect of the CARMA-CBG extract

This example demonstrates "in vivo" the anti-angiogenic effects of the present inventive method by illustrating that oral treatment with CARMA-CBG extract greatly prevents angiogenesis in mice.

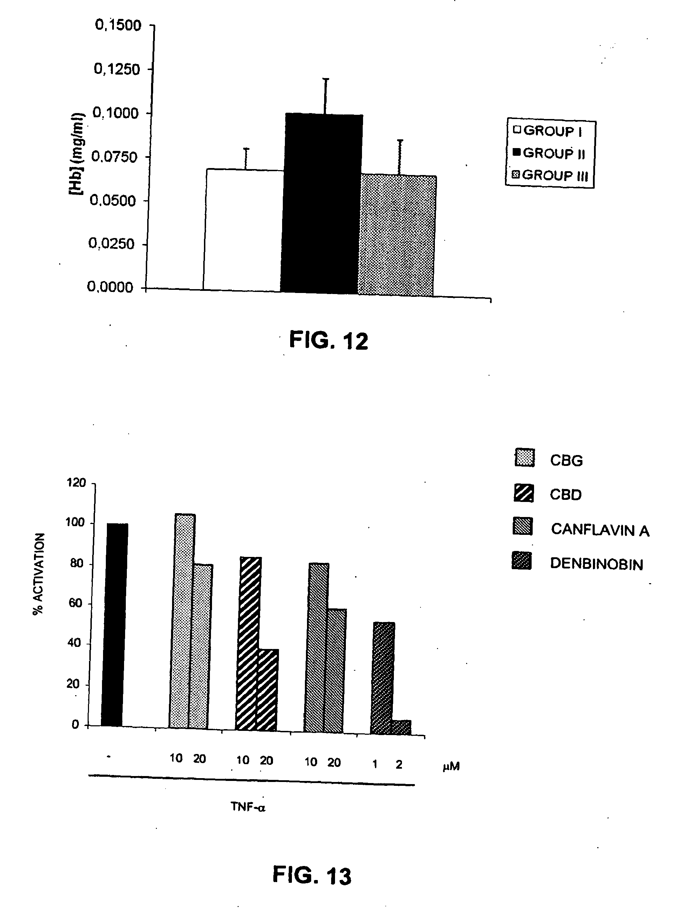

**Methods.** Eight weeks old female Balb/c mice were divided into four groups with ten mice per group. The day prior to the experiment a frozen 10ml bottle of Matrigel was put at 4°C to thaw it. At the experiment day, 30µl of Heparin 16000U/ml and 30µl of aFGF 0'25µg/ml were mixed with 7'5ml of Matrigel at 4°C. Another 2'5ml of Matrigel at 4°C were mixed with 10µl of Heparin 16000U/ml and 10µl of sterile PBS, to inject control animals (without aFGF). Every mouse was subcutaneously injected with 250µl of the mixture using a syringe with 25 3/8-G needle. The injection was done in the rib cage, close to the sternum, but well below the axillary lymph nodes. Three groups were injected with Matrigel/aFGF/Heparin mixture and one group (used as an internal control) injected with the Matrigel/Heparin/PBS preparation. Next day orally treatment with placebo or Cannabis extracts was started. During five days the experimental animals were treated with Placebo -Cremophor 10%- (group I and II) and Cannabis extract CBG (group III, 100 mg/Kg). After five days of daily treatment, animals were euthanized with CO₂ and the gel was extracted using scissors and forceps. The extracted matrigel was homogenized in 9 volumes of PBS employing a homogenizer. 15 µl from each sample was dissolved in 100 µl of 90% glacial acetic acid and let stand for at least 20 mins. 10 µl from each sample and from haemoglobin standards (0'6, 0'3, 0'15, 0'075, 0'0375, 0'0188 and 0'009mg/ml) were added to a 96-well plate containing 140 µl of TMB 5mg/ml. Finally, 150 µl of 0.3% hydrogen peroxide were added to the plate and mix. Using a plate reader, the changes in absorbance at 600nm (550nm) were measured and calculated the concentration of haemoglobin in the samples by comparing to the standards (Figure 12).

### EXAMPLE 13: Inhibition of the TNF-α induced activation of the transcription factor kB (NF-kB) by either Cannabigerol (CBG), or Cannabidiol (CBD), or Canflavin A or Denbinobin in lymphoid cells.

To determine NF-kB-dependent transcription of the HIV-1-LTR-luc, 5.1 cells were preincubated for 30 min with either CBG, or CBD, or Canflavin A, or Denbinobin, as indicated, followed by stimulation with TNF- α for 6 h, and luciferase activity was measured, as described above. Protein concentration was determined by Bradford method (Bio-Rad, Richmond, CA). The results are shown as the percentage of activation (considering the TNF-α treated alone cells 100% activation). Results represent mean ± SD of three different experiments. By using the 5.1 stably transfected cell line it was shown that Denbinobin, potently inhibited TNF- α -induced HIV-1-LTR transactivation, in a concentration-dependent manner, followed by CBD, Canflavin A and CBG (Figure 13).

### EXAMPLE 14. Additive or synergy effects produced on inhibition of the TNF-α induced activation of the transcription factor kB (NF-kB) by either Cannabigerol (CBG), or Cannabidiol (CBD), or Canflavin A or Denbinobin combined with either CBD, or Canflavin A or Denbinobin in lymphoid cells.

Either Cannabigerol (CBG), or Cannabidiol (CBD), or Canflavin A or Denbinobin were tested in combinations, as indicated, and analysed in a NF-kB dependent luciferase gene reporter assay, where the amount of luciferase gene's product reflects the extent of NF-kB transcriptional activation.

Comparing to the results obtained with the compounds tested alone, it was shown that the combination of this compounds in pairs, had an additive or synergy effect.

CBG had an additive effect combined either with CBD or Denbinobin, but synergized when combined with either CBD, or Canflavin A, or Denbinobin, in the indicated concentrations.

CBD had an additive effect or synergized with either Canflavin A, or Denbinobin, depending on the concentrations tested.

Canflavin A had an additive effect when combined with Denbinobin at all tested concentrations.

**Table 2**

| **%INHIBITON OF TNF-α-INDUCED NF-κB ACTIVATION** | | **CBD** | | **CBG** | | **CANFLAVIN A** | | **DENBINOBIN** | |
|---|---|---|---|---|---|---|---|---|---|
| | | **10**µ**M** | **20**µ**M** | **10**µ**M** | **20**µ**M** | **10**µ**M** | **20**µ**M** | **1**µ**M** | **2**µ**M** |
| | | 14,43 | 59,67 | 0,00 | 18,33 | 16,62 | 38,83 | 44,88 | 93,07 |
| **CBD** | **6** µ**M** | | | | | | | | |
| | **10** µ**M** | | | | | | | | |
| | **25** µ**M** | | | | | | | | |
| **CBG** | **5** µ**M** | -4,90 | | | | 83,86 * | | | |
| | **10** µ**M** | 20,07 * | | 85,71 ** | | | | | |
| | **25** µ**M** | 79,01 ** | 94,47 ** | | | | | | |
| **CANFLAVIN A** | **5** µ**M** | -31,59 | 67,48 * | 0,22 | 17,45 | | | | |
| | **10** µ**M** | 27,33 | 88,25 ** | 28,29 ** | 33,40 | | | | |
| | **26** µ**M** | 89,52 ** | 99,40 * | 69,44 ** | 87,50 ** | | | | |
| **DENBINOBIN** | **0,25** µ**M** | 58,07 ** | 91,15 ** | 18,08 | 16,03 | 44,96 * | 56,83 * | | |
| | **0,5** µ**M** | 71,93 ** | 96,15 ** | 25,12 | 50,07 ** | 52,34 * | 74,44 * | | |
| | **1** µ**M** | 88,17 ** | 98,59 * | 59,10 | 64,72 * | 74,41 * | 88,05 * | | |
| | **2** µ**M** | 100,00 * | 100,00 * | 91,79 | 97,21 * | 100,00 * | 100,00 * | | |

| | | | | | | | | | |
|---|---|---|---|---|---|---|---|---|---|
| *** ADDITIVE EFFECTS** **** SYNERGY EFFECTS** | | | | | | | | | |

**Table 2** shows the synergistic or additive effects of Cannabigerol, Cannabidiol, Denbinobin and Canflavin-A on the inhibition of TNFα-induced NF-KB activation.

### EXAMPLE 15. Inhibition of the LPS- induced production of the proinflammatory cytokine IL-1 by either Cannabigerol (CBG), or Cannabidiol (CBD), or Denbinobin in monocytes.

Monocytes were isolated from healthy human donors as indicated above, and then treated with LPS (10 ng/ml), in the absence or presence of either CBG, or CBD or Denbinobin, as indicated, dissolved in DMSO. The production of IL-1 was determined by ELISA, and mean values from two independent experiments are shown. Full LPS-mediated activation was arbitrarily set as 100%.

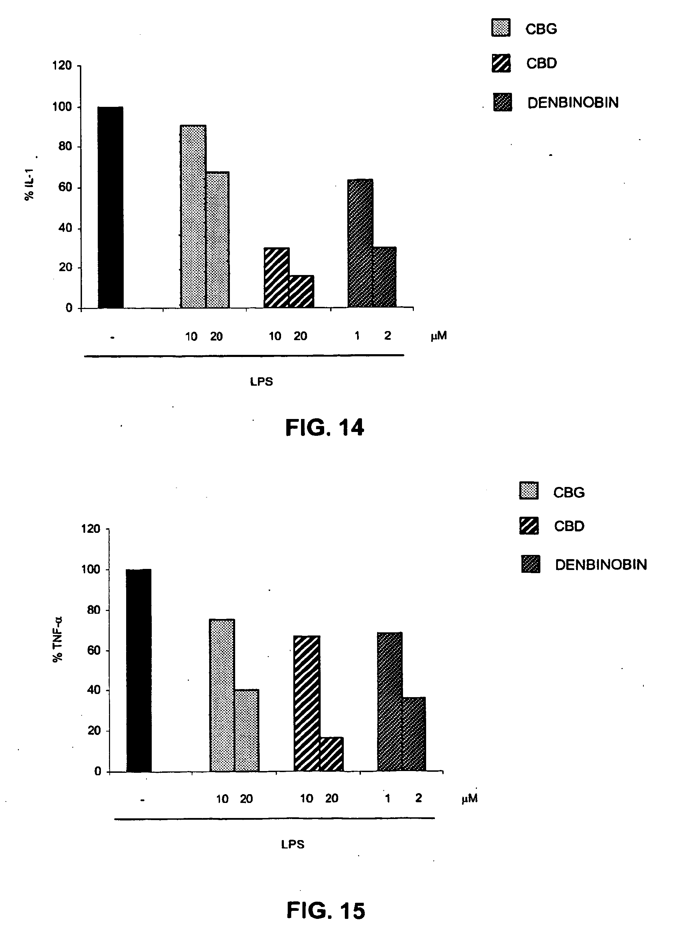

The different compounds caused a significant concentration-dependent decrease of LPS-induced IL-1 secretion into the media, being the most potent the Denbinobin, followed by CBD and CBG (Figure 14)

### EXAMPLE 16. Additive or synergy effects produced on the inhibition of LPS- induced production of proinflammatory cytokine IL-1, by either Cannabigerol (CBG), or Cannabidiol (CBD), or Canflavin A or Denbinobin combined with either CBD, or Canflavin A or Denbinobin in monocytes.

Monocytes from healthy human donors, were treated with LPS (10 ng/ml), in the absence or presence of either CBG, or CBD or Canflavin A or Denbinobin, combined as indicated. The production of IL-1 was determined by ELISA, and mean values from two independent experiments are shown.

Results were compared to those obtained with the compounds tested alone, and it was shown that the combination of these compounds in pairs, had an additive or synergy effect depending on the concentrations of the compounds employed in each case.

CBG had an additive effect combined either with CBD or Denbinobin, but synergized when combined with either Canflavin A, or Denbinobin, in the indicated concentrations.

CBD had an additive effect combined either with Canflavin A, or Denbinobin but synergized with Canflavin A depending on the concentrations tested.

Canflavin A synergized when combined with Denbinobin.

**Table 3**

| **%INHIBITION OF LPS-INDUCED IL-1 PRODUCTION** | | **CBD** | | **CBG** | | **CANFLAVIN A** | | **DENBINOBIN** | |
|---|---|---|---|---|---|---|---|---|---|
| | | **10**µ**M** | **20**µ**M** | **10**µ**M** | **20**µ**M** | **10**µ**M** | **20**µ**M** | **1**µ**M** | **2**µ**M** |
| | | 70,10 | 84,00 | 9,40 | 32,60 | 0,00 | 0,00 | 37,00 | 70,00 |
| **CBD** | **5** µ**M** | | | | | | | | |
| | **10** µ**M** | | | | | | | | |
| | **25** µ**M** | | | | | | | | |
| **CBG** | **1** µ**M** | 68,78 | 91,44 * | | | | | | |
| | **5** µ**M** | 82,71 | 93,43 * | | | | | | |
| | **10** µ**M** | 91,32 ** | 98,52 * | | | | | | |
| | **25** µ**M** | 100,00 * | 100,00 * | | | | | | |
| **CANFLAVIN A** | **1** µ**M** | 89,27 ** | 89,53 | 21,45 * | 84,70 ** | | | | |
| | **5** µ**M** | 40,51 | 92,04 * | 55,81 ** | 69,63 ** | | | | |
| | **10** µ**M** | 47,47 | 96,01 ** | 77,32 ** | 61,05 | | | | |
| | **25** µ**M** | 68,51 | 100,00 ** | 85.00 ** | 46.54 | | | | |
| **DENBINOBIN** | **0,25** µ**M** | | 92,94 * | | 51,43 * | | | | |
| | **0,5** µ**M** | | 100,00 * | | 81,94 ** | | | | |
| | **1** µ**M** | 95,41 * | 100,00 * | 40,12 | 100,00 * | -8,06 | 38,03 * | | |
| | **2** µ**M** | 100.00 * | 100,00 * | 100,00 ** | 100,00 * | 100,00 ** | 96,49 ** | | |

| | | | | | | | | | |
|---|---|---|---|---|---|---|---|---|---|
| *** ADDITIVE EFFECTS** **** SYNERGY EFFECTS** | | | | | | | | | |

**Table 3** shows the synergitic or additive effect of Canabigerol, Cannabidiol, Denbinobin and Canflavin-A on LPS-induced IL-1β release,

### EXAMPLE 17. Inhibition of LPS- induced production of the proinflammatory cytokine TNF-α by either Cannabigerol (CBG), or Cannabidiol (CBD), or Denbinobin in monocytes.

The production of TNF-α was determined by ELISA, on the supernatant of LPS-treated monocytes, as indicated above, in the absence or presence of either CBG, or CBD or Denbinobin, as indicated.

The different compounds caused a significant concentration-dependent decrease of LPS-induced TNF-α secretion in to the media, being the most potent the Denbinobin, followed by CBD and CBG (Figure 15)

### EXAMPLE 18. Additive or synergy effects produced on the inhibition of LPS- induced production of the proinflammatory cytokine TNF-α, by either Cannabigerol (CBG), or Cannabidiol (CBD), or Canflavin A or Denbinobin combined with either CBD, or Canflavin A or Denbinobin in monocytes.

Monocytes from healthy human donors, were treated with LPS (10 ng/ml), in the absence or presence of either CBG, or CBD or Canflavin A or Denbinobin, combined as indicated. The production of TNF-α was determined by ELISA as indicated above.

Results were compared to those obtained with the compounds tested alone, and it was shown that the combination in pairs, had an additive or synergy effect depending on the concentrations of the compounds used in each case.

CBG had an additive effect combined either with CBD or Denbinobin, but synergized when combined with either Canflavin A, or Denbinobin, in the indicated concentrations.

CBD had an additive effect combined either with Canflavin A, or Denbinobin but synergized with Canflavin A depending on the concentrations tested.

**Table 4**

| **%INHIBITION OF LPS-INDUCED TNF-α-PRODUCTION** | | **CBD** | | **CBG** | | **CANFLAVIN A** | | **DENBINOBIN** | |
|---|---|---|---|---|---|---|---|---|---|
| | | **10**µ**M** | **20**µ**M** | **10**µ**M** | **20**µ**M** | **10**µ**M** | **20**µ**M** | **1**µ**M** | **2**µ**M** |
| - | | 33,30 | 83,60 | 24,90 | 59,80 | 0,00 | 0,00 | 32,00 | 64,00 |
| **CBD** | **5** µ**M** | | | | | | | | |
| | **10** µ**M** | | | | | | | | |
| | **25** µ**M** | | | | | | | | |
| **CBG** | **1** µ**M** | 17,05 | 88,60 | | | | | | |
| | **5** µ**M** | 24,35 | 100,00 * | | | | | | |
| | **10** µ**M** | 72,45 | 100,00 * | | | | | | |
| | **25** µ**M** | 100,00 | 100,00 * | | | | | | |
| **CANFLAVIN A** | **1** µ**M** | 6,20 | 98,28 * | -43,68 | -26,74 | | | | |
| | **5** µ**M** | 17,89 | 98,91 * | 18,40 | -6,49 | | | | |
| | **10** µ**M** | 29,01 | 100,00 ** | 34,79 | 28,70 | | | | |
| | **25** µ**M** | 66,66 ** | 100,00 ** | 57,94 ** | 48,25 | | | | |
| **DENBINOBIN** | **0,25** µ**M** | | 99,82 | | 76,99 ** | | | | |
| | **0,5** µ**M** | | 100,00 * | | 92,12 ** | | | | |
| | **1** µ**M** | 98,78 ** | 100,00 * | 86,31 ** | 100,00 * | 80,25 ** | 64,64 ** | | |
| | **2** µ**M** | 100,00 * | 100,00 * | 100,00 ** | 100,00 * | 100,00 ** | 95,04 ** | | |

| | | | | | | | | | |
|---|---|---|---|---|---|---|---|---|---|
| *** ADDITIVE EFFECTS** ****SYNERGY EFFECTS** | | | | | | | | | |

**Table 4** shows the synergistic or additive effects of Cannabigerol, Cannabidiol, Denbinobin and Canflavin-A on LPS-induced TNF-1α release.

### EXAMPLE 19. Additive cytotoxic activities of Cannabigerol (CBG) and Cannabidiol (CBD) in the gastrointestinal cancer cell lines AGS, HCT-116 and SW480.

This example demonstrates the in vitro anti-tumoral effects of the present inventive method by illustrating the cytotoxic effects of the combination between Cannabidiol (CBD) and Cannabigerol (CBG) on the AGS (gastric cancer); HCT-116 and SW480 (Colon cancer) tumoral cell lines.

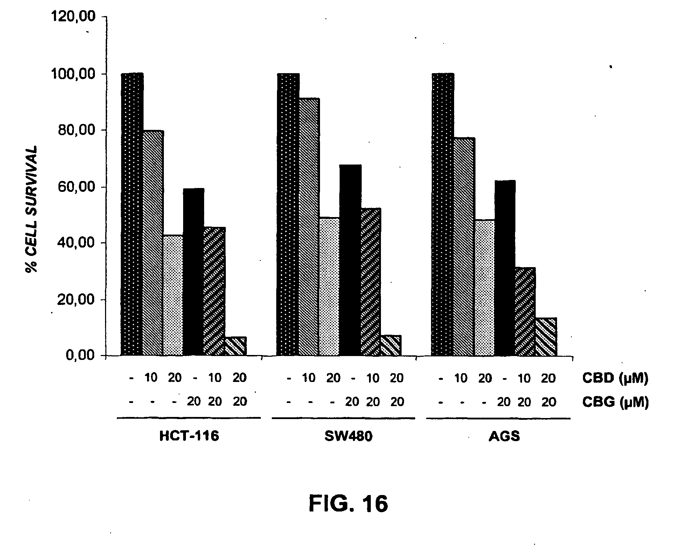

The cytotoxic effect of the Cannabigerol (CBG) and Cannabidiol (CBD) alone or in combination was investigated by the Calcein-AM assay. Briefly, AGS, SW480 and HTC116 cells were cultivated at a density of 1.0 × 10⁴ cells/ml in 96-well plates, 200 µl cell suspension per well and cultured in DMEM containing 10% fetal calf serum. Cells were treated with or without CBD, CBG or a combination of both compounds for 24 h, after which the wells were washed and the cells incubated with Calcein-AM (1 µM) (Molecular Probes) for 30 min. Then the fluorescence of viable cells was detected in a microtitre plate reader (TECAN Genius Pro). The intensity of fluorescence was inversely proportional to cell death induced by the compounds. The results are represented as the percentage of cell survival (given the value of 100% of survival to control untreated cells). Both CBD and CBG induced cytotoxicity in the three cell lines and an additive effect was observed when the cells were treated with a combination of both compounds (Figure 16).

## Claims

1. A composition comprising cannabidiol and denbinobin, and having a content of delta-9-tetrahydrocannabidiol less than 0.7% of the total weight of the composition, wherein
a. denbinobin is present at concentrations between 0.25 µM and 0.5 µM when cannabidiol is present at concentrations between 10 µM and 20 µM, and
b. denbinobin is present at concentrations between 0.25 µM and 1 µM when cannabidiol is present at concentrations of 10 µM.

2. The composition according to claim 1, wherein the composition is a cannabis-based composition.

3. The composition according to anyone of claims 1-2, wherein the cannabidiol and denbinobin are derived from at least one extract from at least one cannabis plant.

4. The composition according to claim 3, wherein the cannabis plant is selected from *Cannabis sativa.*

5. The cannabis-based composition of claim 4, wherein the cannabis plant is selected from the variety CARMA of *Cannabis sativa.*

6. The composition according to anyone of claims 1-5 for use as a medicament.

7. The composition according to anyone of claims 1-5 for use as anti-inflammatory.

8. The composition according to claim 7 for use in the treatment and/or prevention of inflammatory diseases.

9. The composition according to anyone of claims 1-5 for use in the treatment and/or prevention of cancer.

## Patentansprüche

1. Eine Zusammensetzung, die ein Cannabidiol und Denbinobin umfasst und einen Gehalt an Delta-9-Tetrahydrocannabidiol von weniger als 0,7 % des Gesamtgewichts der Zusammensetzung aufweist, wobei
a. Denbinobin in Konzentrationen von 0,25 µM bis 0,5 µM und Cannabidiol in Konzentrationen von 10 µM bis 20 µM vorhanden ist, und
b. Denbinobin in Konzentrationen von 0,25 µM bis 1 µM vorhanden ist, wenn Cannabidiol in einer Konzentrationen von 10 µM vorhanden ist.

2. Die Zusammensetzung nach Anspruch 1, wobei die Zusammensetzung eines Cannabis-basierte Zusammensetzung ist.

3. Die Zusammensetzung nach einem der Ansprüche 1-2, wobei das Cannabidiol und das Denbinobin aus mindestens einem Extrakt von mindestens einer Cannabis-Pflanze abgeleitet sind.

4. Die Zusammensetzung nach Anspruch 3, wobei die Cannabis-Pfianze aus der Pflanzenart *Cannabis sativa* ausgewählt ist.

5. Die Cannabis-basierte Zusammensetzung nach Anspruch 4, wobei die Cannabis-Pflanze aus der Varietät CARMA der Pflanzenart *Cannabis sativa* ausgewählt ist.

6. Die Zusammensetzung nach einem der Ansprüche 1-5 zur Verwendung als Medikament.

7. Die Zusammensetzung nach einem der Ansprüche 1-5 zur Verwendung als Entzündungshemmer.

8. Die Zusammensetzung nach Anspruch 7 zur Verwendung bei der Behandlung und/oder Prävention von inflammstorischen Krankheiten.

9. Verwendung der Zusammensetzung nach einem der Ansprüche 1-5 zur Verwendung bei der Behandlung und/oder Prävention von Krebs.

## Revendications

1. Une composition comprenant cannabidiol et denhinobin, et ayant une teneur en delta-9-tétrahydrocannahidiol inférieure à 0,7% du poids total de la composition, dans laquelle
a. denbinobin est présent à des concentrations allant de 0,25 µM à 0,5 µM lorsque le cannabidiol est présent à des concentrations allant de 10 µM à 20 µM, et
b. denbinobin est présent à des concentrations allant de 0,25 µM à 1 µM lorsque le est présent à des concentrations de 10 µM.

2. La composition selon la revendication 1, dans laquelle la composition est une composition à base de cannabis.

3. La composition selon l'une quelconque des revendications 1-2, dans laquelle cannabidiol et denbinobin sont dérivés d'au moins un extrait provenant d'au moins une plante de cannabis.

4. La composition selon la revendication 3, dans laquelle la plante de cannabis est sélectionnée parmi des *Cannabis sativa*.

5. La composition à base de cannabis de la revendication 4, dans laquelle la plante de cannabis est sélectionnée dans la variété CARMA de *Cannabis sativa*.

6. La composition selon l'une quelconque des revendications 1-5 à utiliser comme un médicament.

7. La composition selon l'une quelconque des revendications 1-5 à utiliser comme un anti-inflammatoire.

8. La composition selon la revendication 7, à utiliser dans le traitement et/ou la prévention de maladies inflammatoires.

9. La composition selon l'une quelconque des revendications 1-5 à utiliser dans le traitement et/ou la prévention du cancer.
